# EUROPEAN PATENT APPLICATION

(11) **EP 0 931 548 A2**
(43) Date of publication of application: **28.07.1999**
(21) Application number: 99300458.9
(22) Date of filing: 21.01.1999
(51) Int. Cl.: A61K 31/70

(54) **Antifibrotic agent**

(30) Priority: 22.01.1998 JP 1064298
(71) Applicant: Seikagaku Corporation, Chuo-ku, Tokyo 103-0023 (JP)
(72) Inventor: Hoshino, Jiro, Ohme-shi, Tokyo 198-0024 (JP); Sekiguchi, Tomoko, Akishima-shi, Tokyo 196-0003 (JP); Yamazaki, Chihiro, Higashiyamato-shi, Tokyo 207-0022 (JP); Asari, Akira, Iruma-shi, Saitama-ken 358-0051 (JP)
(74) Representative: Bannerman, David Gardner

(57) **Abstract**

An antifibrotic agent comprising a keratan sulfate oligosaccharide or a pharmaceutically acceptable salt thereof as an active ingredient. The antifibrotic agent is used as a prophylactic or a depressant for fibrosis accompanying one or more diseases preferably selected from the group consisting of pulmonary fibrosis, hepatocirrhosis, arteriosclerosis, scleroderma, restenosis of coronary artery after percutaneous transluminal coronary angioplasty (PTCA), interstitial myocarditis, interstitial cystitis, glomerulonephritis, angiitis, diabetic nephropathy, hypertensive nephrosclerosis, HIV nephropathy, IgA nephropathy, lupus nephritis, interstitial nephritis, obstructed kidney due to ureteral obstruction, skin scar formation after burn, and toxicosis.

## Description

### BACKGROUND OF THE INVENTION

The present invention relates to a medical use of a keratan sulfate oligosaccharide and more particularly to an antifibrotic agent containing a keratan sulfate oligosaccharide as an active ingredient.

In chronic inflammations such as interstitial pneumonia and hepatocirrhosis, there occurs increase of production and secretion of active oxygen and proliferating cytokine from inflammatory cells due to certain stimulation, resulting in proliferation of fibroblasts and overproduction of extracellular matrix proteins, to lead to irreversible fibrosis. while chronic inflammations have been given treatment with antiinflammatory agents such as steroid agents or the like, currently no drug is available that depresses the progress of fibrogis, so that an effective therapeutical method is needed.

Also, administration of antineoplastic agents may cause pulmonary fibrosis or the like as a side-effect and a drug that effectively prevents/suppresses such a side-effect (fibrosis) are needed.

### SUMMARY OF THE INVENTION

As described above, there is a demand for a drug that prevents/suppresses fibrosis. Therefore, an object of the present invention is to provide a drug that prevents fibrosis and depresses progress of fibrosis, that is, an antifibrotic agent.

As a result of intensive investigation with view to solving the above-described problems, the present inventors have found that keratan sulfate oligosaccharides that have been known to be useful as an active ingredient of an antiinflammatory agent, an antiallergic agent, an immunomodulator, a differentiation inducer, and an apotosis inducer (International Publication No. WO96/16973), have an antifibrotic effect, thus completing the present invention.

Thus, the present invention is to provide an antifibrotic agent comprising a keratan sulfate oligosaccharide or a pharmaceutically acceptable salt thereof as an active ingredient (hereafter, also referred to as "the antifibrotic agent of the present invention").

The antifibrotic agent of the present invention is used as a prophylactic or depressant of fibrosis accompanying one or more diseases preferably selected from the group consisting of pulmonary fibrosis, hepatocirrhosis, arteriosclerosis, scleroderma, restenosis of coronary artery after percutaneous transluminal coronary angioplasty (PTCA), interstitial myocarditis, interstitial cystitis, glomerulonephritis, angiitis, diabetic nephropathy, hypertensive nephrosclerosis, HIV nephropathy, IgA nephropathy, lupus nephritis, interstitial nephritis, obstructed kidney due to ureteral obstruction, skin scar formation after burn, and toxicosis.

Preferably, the antifibrotic agent of the present invention is used against fibrosis caused by administration of a drug. The drug is preferably an antineoplastic agent, an antibiotic, an antibacterial agent, an antiarrhythmic agent, an antiphlogistic agent, an antirheumatic agent, an interferon, or shosaikoto.

More preferably, the antifibrotic agent of the present invention is a prophylactic or a depressant for pulmonary fibrosis accompanying the administration of an antineoplastic agent. The antineoplastic agent is preferably an antineoplastic antibiotic.

The antifibrotic agent of the present invention may be provided as an antifibrotic composition comprising an effective amount of the keratan sulfate oligosaccharide or the pharmaceutically acceptable salt thereof and a pharmaceutically acceptable carrier.

The present invention also provides a method for preventing or depressing fibrosis, comprising the step of administering an effective amount of a keratan sulfate oligosaccharide or a pharmaceutically acceptable salt thereof to a subject, or use of the keratan sulfate oligosaccharide or the pharmaceutically acceptable salt thereof for the manufacture of the antifibrotic agent.

### BRIEF EXPLANATION OF THE DRAWINGS

Fig. 1 is a graph illustrating the vet weights of lungs of mice to which keratan sulfate tetrasaccharide (L4L4) was administered at various doses.
Fig. 2 is a graph illustrating the total cell number in bronchoalveolar lavage fluids (BALF) of mice to which keratan sulfate tetrasaccharide was administered at various doses.
Fig. 3 is a graph illustrating the protein content in bronchoalveolar lavage fluids of mice to which keratan sulfate tetrasaccharide was administered at various doses.
Fig. 4 is a graph illustrating the hydroxyproline content in lung of mice to which keratan sulfate tetrasaccharide was administered at various doses.
Fig. 5 is a graph illustrating the pulmonary fibrosis scores of mice to which keratan sulfate tetrasaccharide was administered at various doses.
Fig. 6 is a graph illustrating the wet weights of lungs of mice to which keratan sulfate disaccharide (L4) was administered at various doses.
Fig. 7 is a graph illustrating the total cell number in bronchoalveolar lavage fluids of mice to which keratan sulfate disaccharide was administered at various doses.
Fig. 8 is a graph illustrating the protein content in bronchoalveolar lavage fluids of mice to which keratan sulfate disaccharide was administered at various doses.
Fig. 9 is a graph illustrating the hydroxyproline content in lung of mice to which keratan sulfate disaccharide was administered at various doses.
Fig. 10 is a graph illustrating the pulmonary fibrosis scores of mice to which keratan sulfate disaccharide was administered at various doses.
Fig. 11 is a set of photographs illustrating normal tissue image (N), minimal change tissue image (MC), and diffuse proliferative tissue image (DP) in the glomerulus.
Fig. 12 is a set of photographs illustrating tissue images of grades 1 to 4 of renal artery.
Fig. 13 is a graph illustrating the blood creatinine levels in mice to which keratan sulfate disaccharide was administered at various doses.
Fig. 14 is a graph illustrating the incidence of MC in the glomerulus of mice to which keratan sulfate disaccharide was administered at various doses.
Fig. 15 is a graph illustrating the incidence of DP in the glomerulus of mice to which keratan sulfate disaccharide was administered at various doses.
Fig. 16 is a graph illustrating the changes in grade of tissue image in the renal artery of mice to which keratan sulfate disaccharide was administered at various doses.
Fig. 17 is a graph illustrating the urine albumin levels in mice to which keratan sulfate disaccharide was administered at various doses.
Fig. 18 is a graph illustrating the ratios of urine albumin level to urine creatinine level in mice to which keratan sulfate disaccharide was administered at various doses.

### DETAILED DESCRIPTION OF THE INVENTION

Hereafter, embodiments of the present invention will be explained in more detail.

The antifibrotic agent of the present invention comprises a keratan sulfate oligosaccharide or a pharmaceutically acceptable salt thereof as an active ingredient.

The keratan sulfate oligosaccharide used in the antifibrotic agent of the present invention is not limited particularly so far as it is an oligosaccharide derived from keratan sulfate. However, it is preferably a degradate of keratan sulfate obtained by degrading keratan sulfate with an endo-β-N-acetylglucosaminidase type keratan sulfate-degrading enzyme.

The keratan sulfate oligosaccharide may contain a sialic acid residue and/or a fucose residue. Usually, the sialic acid residue is linked to a galactose residue through an α-2,3- or α-2,6-glycosidic linkage while the fucose residue is linked to a galactose residue through an α-1,3-glycosidic linkage.

Preferably, the keratan sulfate oligosaccharide includes di- to penta-saccharides having a sulfated N-acetylglucosamine residue at the reducing end, in which the hydroxyl groups at at least two positions of each molecule have been sulfated, particularly those keratan sulfate oligosaccharides described above that contain, as a constituent unit, at least a disaccharide represented by Gal(6S)-GlcNAc(6S) wherein Gal represent a galactose residue, GlcNAc represents an N-acetylglucosamine residue, and 6S represents a 6-O-sulfate group.

More preferably, the above-described keratan sulfate oligosaccharide is selected from a tetrasulfated N-acetyllactosamine tetrasaccharide represented by the formula (I) and a disulfated N-acetyllactosamine disaccharide represented by the formula (II):

Gal(6S)β1-4GlcNAc(6S)β1-3Gal(6S)β1-4GlcNAc(6S) (I)

Gal(6S)β1-4GlcNAc(6S) (II)

wherein, Gal represents a galactose residue, GlcNAc represents an N-acetylglucosamine residue, and 6S represents a 6-O-sulfate group.

The keratan sulfate oligosaccharide may comprise a single species or mixtures of two or more species.

The keratan sulfate oligosaccharide used in the present invention can be obtained by allowing an endo-β-N-acetylglucosaminidase type keratan sulfate-degrading enzyme, for example, keratanase II derived from a bacterium belonging to the genus Bacillus (Japanese Patent Application Laid-open No. 2-57182 (1990)) or a keratan sulfate-degrading enzyme derived from Bacillus circulans KsT202 (International Publication No. WO96/16166) to act on a buffered solution of keratan sulfate, preferably highly sulfated keratan sulfate, to degrade the keratan sulfate, and then fractionating the resulting degradate. The oligosaccharide thus obtained may be subjected to ordinary purification including concentration by precipitation with ethanol, gel filtration and anion exchange chromatography to obtain a targeted oligosaccharide.

The keratan sulfate used as a starting material for the keratan sulfate oligosaccharide used in the present invention is mainly composed of a disaccharide repeating structure of galactose or galactose-6-sulfate and N-acetylglucosamine-6-sulfate. The sulfate content may vary depending on the animal species and organ. Usually, it is produced from raw materials such as cartilage, bones, and cornea of cartilaginous fish, e.g., shark, mammals, e.g., whale and oxen and the like.

The keratan sulfate used as a starting material is not limited particularly and may be any usually available one. However, it is preferred to use highly sulfated keratan sulfate, in which the galactose residues, which is constituent sugars, have been sulfated (highly sulfated keratan sulfate that contains sulfate groups of 1.5 to 2 molecules per constituent disaccharide may be referred to as "keratan polysulfate"). The sulfate is bonded to a galactose residue preferably at the 6-position. Such a highly sulfated keratan sulfate can be obtained from the proteoglycan of cartilaginous fish such as shark. Alternatively, commercially available ones may be used.

The keratan sulfate oligosaccharide used in the present invention may include that in an ionized state, and that having a structure in which one or more protons have been added. As the pharmaceutically acceptable salt can be used, for example, a salt among salts with inorganic bases such as alkali metal salts such as sodium salt, potassium salt and lithium salt, alkaline earth metal salts such as calcium salt, salts with inorganic bases such as ammonium salt, and salts with organic bases such as diethanolamine salt, cyclohexylamine salt and amino acid salt, which is pharmaceutically acceptable. However, it is not limited thereto.

The keratan sulfate oligosaccharide obtainable as described above can be formulated as an active ingredient in dosage forms described hereinbelow to form a desired antifibrotic agent.

The disease to which the present invention is applicable includes diseases accompanied by fibrosis in mammals including humans, and the antifibrotic agent of the present invention can be administered to subjects for therapy as a prophylactic or a depressant for the fibrosis accompanying the diseases. The prophylactic or the depressant may be administered with a view to preventing, or depressing the progress of (i.e., maintaining) the fibrosis accompanying the diseases.

Specific examples of the diseases include pulmonary fibrosis, hepatocirrhosis, arteriosclerosis, scleroderma, restenosis of coronary artery after percutaneous transluminal coronary angioplasty (PTCA), interstitial myocarditis, interstitial cystitis, glomerulonephritis, angiitis, diabetic nephropathy, hypertensive nephrosclerosis, HIV nephropathy, IgA nephropathy, lupus nephritis, interstitial nephritis, obstructed kidney due to ureteral obstruction, skin scar formation after burn and toxicosis (e.g., paraquat poisoning). Among the diseases, one particularly suitable for the application of the antifibrotic agent of the present invention is fibrosis accompanying pulmonary fibrosis, glomerulonephritis, or angiitis.

Further, the antifibrotic agent of the present invention is also effective to fibrosis caused by the administration of drugs. Those drugs that may possibly cause fibrosis include, for example, antineoplastic agents, antibiotics, antibacterial agents, antiarrhythmic agents, antiphlogistic agents, antirheumatic agents, interferons, and shosaikoto. The antifibrotic agent of the present invention can be applied more preferably to the fibrosis caused by the administration of these drugs.

In particular, the antifibrotic agent of the present invention is preferably a prophylactic or a depressant for fibrosis accompanying the administration of antineoplastic agents and more preferably a prophylactic agent or a depressant for pulmonary fibrosis accompanying the administration of an antineoplastic agent. Examples of the antineoplastic agent include bleomycin, methotrexate, procarbazine, melphalan, busulfan, mitomycin C, fluorouracil, Vinca alkaloids, azathioprine, cytosine arabinoside, cyclophosphamide, chlorambucil, nitrosourea compounds, etoposide, zinostatin, and the like. The diseases to which the antifibrotic agent of the present invention is applied particularly advantageously are pulmonary fibrosis, particularly interstitial pulmonary diseases (especially, interstitial pneumonia) caused by administration of antineoplastic antibiotics, particularly bleomycin among the antineoplastic agents.

However, the above-listed diseases are only examples and the scope of application of the keratan sulfate oligosaccharide preparations should not be limited to such exemplary diseases.

The antifibrotic agent of the present invention may contain, together with the keratan sulfate oligosaccharide, one or more substances other than the keratan sulfate oligosaccharide, which have an antifibrotic activity, as an active ingredient.

The "substance other than the keratan sulfate oligosaccharide, which has an antifibrotic activity" is not limited particularly unless it causes any serious side-effect or one of the active ingredients inhibits the antifibrotic activity that the other substance has inherently when formulated or administered with the keratan sulfate oligosaccharide.

The antifibrotic agent of the present invention may be formulated or administered with an antiinflammatory agent. Examples of a usable antiinflammatory agent include steroid agents such as prednisolone and antiinflammatory enzymes such as superoxide dismutase. However, it is not limited thereto.

Since the antifibrotic agent of the present invention is effective to the fibrosis caused by the administration of the drug, it may be formulated or administered with a drug that may cause fibrosis (for example, the above-described drugs). of course, it is possible to administer the antifibrotic agent of the present invention in advance before the administration of a drug that may cause fibrosis (for example, the above-described drugs).

In the present invention, any dosage form may be selected suitably as a dosage form of the antifibrotic agent of the present invention depending on the nature and progress of the target disease, the method of administration and the like.

That is, the antifibrotic agent of the present invention may be administered by injection (intravenous, intramuscular, subcutaneous, intradermal, intraperitoneal, etc.), oral administration, percutaneous administration, inhalation, or the like and can be prepared appropriately depending on the selected method of administration.

The dosage form that can be selected is not limited particularly and may be selected from a wide range including, for example, injectable preparations (solutions, suspensions, emulsions, solids to be solved when used, etc.), tablets, capsules, granules, powders, liquids, liposome inclusions, ointments, gels, external powders, sprays, inhalating powders and the like. Upon formulating the preparation, there may be used conventional excipients, stabilisers, binders, lubricants, emulsifiers, osmotic pressure-adjusting agents, pH-adjusting agents, and other components such as colorants, disintegrants, and the like usually used in pharmaceuticals (pharmaceutically acceptable carriers).

The formulating amount of the keratan sulfate oligosaccharide, which is an active ingredient of the antifibrotic agent of the present invention and dose of the antifibrotic agent of the present invention should be determined severally depending on the administration method, administration form, and object of use of the preparation concerned as well as specific conditions of the patient, the body weight of the patient, etc. Clinical daily dosage of the keratan sulfate oligosaccharide may be, although not limited particularly thereto, about 300 to 1,500 mg/human/day. The preparation may be administered at intervals of once a day or so or twice or thrice a day in portions.

According to the present invention, there is provided an agent that prevents fibrosis or depresses its progress, i.e., an antifibrotic agent. Since it is effective against the fibrosis caused by the administration of drugs, the antifibrotic agent of the present invention may also be used as a preventive/suppressive agent for the side-effects caused by the administration of the drugs.

### EXAMPLES

Hereafter, the present invention will be explained concretely by test examples and preparation examples. However, the scope of the present invention should not be construed as being limited thereto.

### Test Example 1: Study of effects on bleomycin-induced murine pulmonary fibrosis (interstitial pneumonia) model

In the drug effect and pharmacological test example 1 were used solutions of sodium salts of keratan sulfate tetrasaccharide represented by the formula (I) (hereafter, referred to L4L4) and keratan sulfate disaccharide represented by the formula (II) (hereafter, referred to L4) as a keratan sulfate oligosaccharide. As to the properties of the L4L4 solution, it was a colorless transparent aqueous solution having a pH of about 6 to 7 and an Osmotic pressure ratio to distilled water of about 1. As to the properties of the solution of L4, it was a colorless transparent aqueous solution having a pH of about 6 to 7 and an osmotic pressure ratio to physiological saline of about 1.

### 〈Experimental Method〉

A model was produced by administering 150 mg/kg of bleomycin to 6-week-old ICR male mice once intravenously. On the day next was started and continued for 13 days everyday the administration of a solution of L4L4 or L4 to the mice (each group consisting of 12 to 15 animals) intramuscularly at dosages of 1, 5, and 25 mg/kg, respectively. After 2 weeks from the administration of bleomycin, the animals were dissected and the right lung was subjected to bronchoalveolar lavage 3 times with physiological saline to obtain bronchoalveolar lavage fluids (BALF). The items of evaluation included the total number of cells and total protein content (protein content) in the bronchoalveolar lavage fluids (BALF), the hydroxyproline content (corresponding to the amount of collagen) in the right lung, the wet weight of the left lung, and pulmonary fibrosis score (pathological observation of tissues). The protein content was determined by use of BCA Protein Assay kit (manufactured by PIERCE) and the hydroxyproline content was measured according to the method of Woessner et al. (Woessner, L.F., 1961, Arch. Biochem. Biophys., 93: 440-447). The pulmonary fibrosis score was obtained according to the method of Yokumoto et al. (Hisao Yokumoto, Katsutoshi Takahashi, Akira Matsuda, Hamao Umezawa, 1983, Gan to Kagaku Ryohou (Cancer and Chemical Therapy), 10: 2550-2557).

### 〈Results〉

### (1) Effects of L4L4

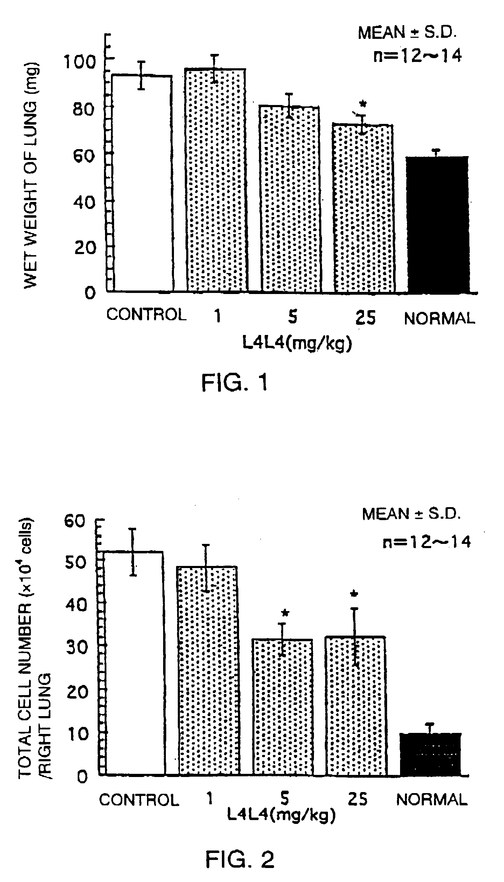

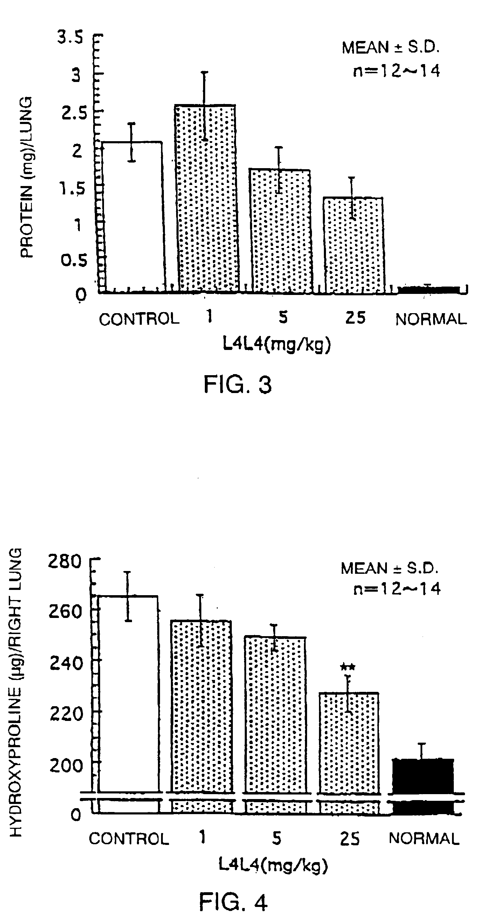

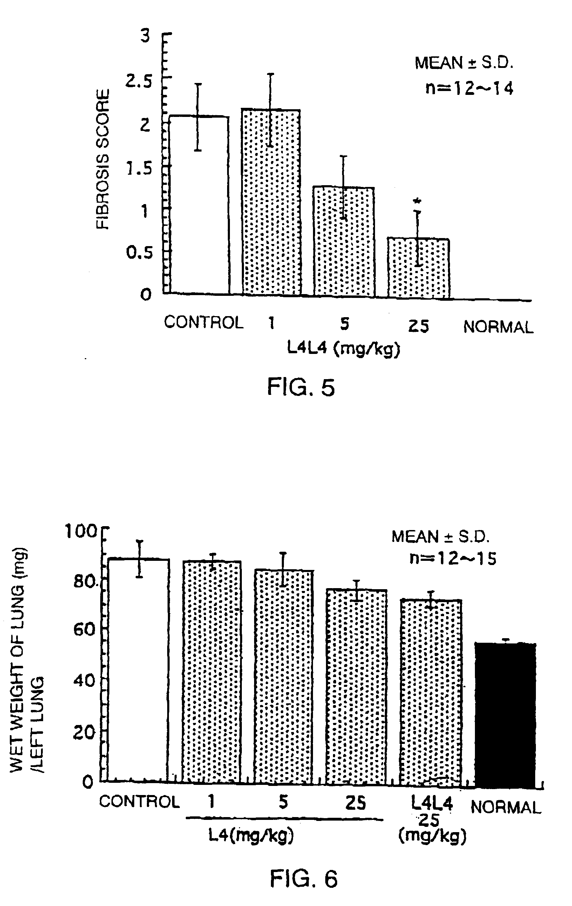

A significant inhibitory effect on the wet weight of lung was observed by administration of 25 mg/kg (Fig. 1). A significant inhibitory effect on the total number of cells in the bronchoalveolar lavage fluids was observed by administration of 5 and 25 mg/kg (Fig. 2). The protein content in the bronchoalveolar lavage fluids showed the lowest value by administration of 25 mg/kg (Fig. 3). Significant inhibitory effects on the hydroxyproline content and pulmonary fibrosis score were observed by administration of 25 mg/kg (Figs. 4 and 5). These results indicate that L4L4 has an antifibrotic activity.

### (2) Effects of L4

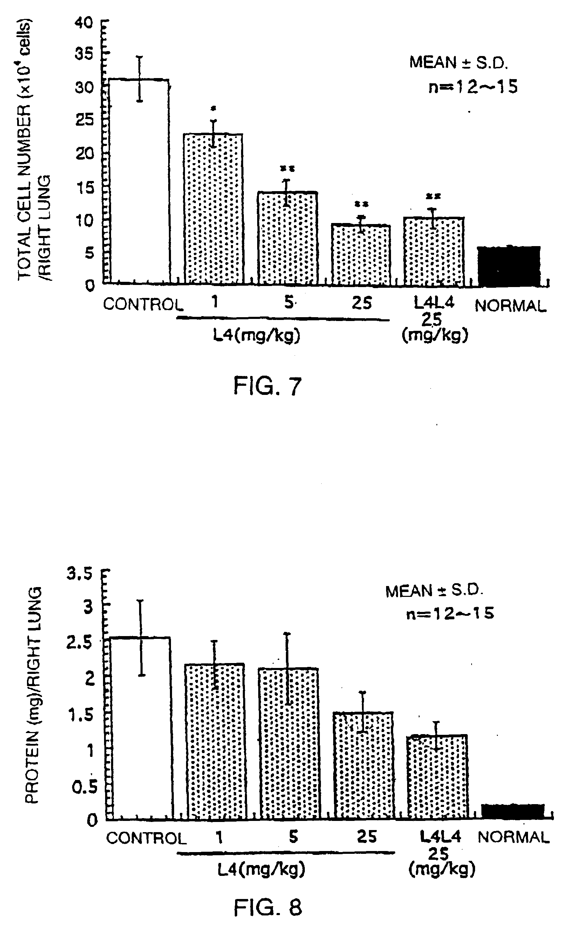

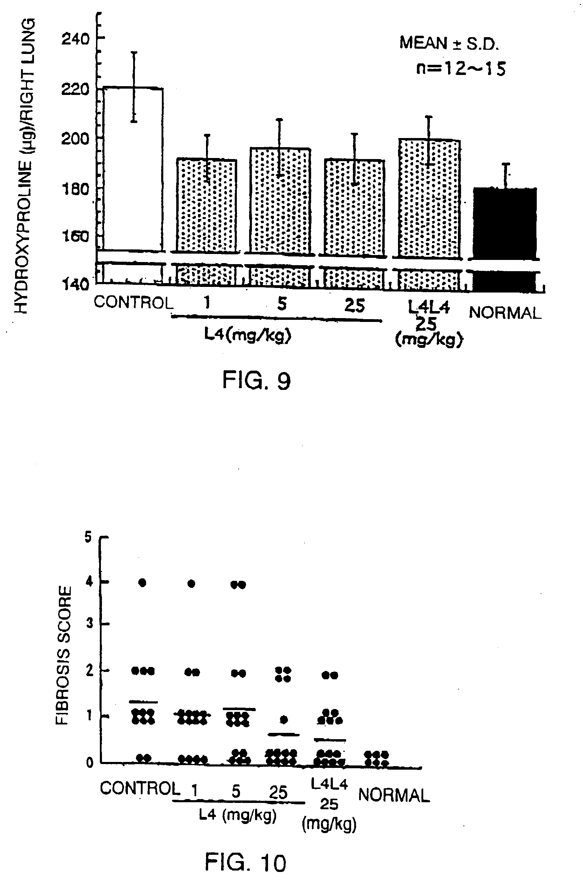

The wet weight of lung showed the lowest value by administration of 25 mg/kg (Fig. 6). A significant inhibitory effect on the total number of cells in the bronchoalveolar lavage fluids was observed by administration of 1, 5, and 25 mg/kg (Fig. 7). The protein content in the bronchoalveolar lavage fluids showed the lowest value by administration of 25 mg/kg (Fig. 8). The hydroxyproline content and pulmonary fibrosis score showed the lowest values by administration of 25 mg/kg (Figs. 9 and 10). These results indicate that L4 has an antifibrotic activity.

Note that in Figs. 1 to 9, the symbol "*" indicates that the values were significant at a level of p<0.05 and the symbol "**" indicates that the values were significant at a level of p<0.01 relative to the control in the multiple comparison test of Dunnett.

### Test Example 2: Study of effects on glomerulonephritis and angiitis in MRL mice

In the drug effect and pharmacological test example 2 was used a solution of sodium salt of keratan sulfate disaccharide represented by the formula (II) (hereafter, referred to L4) as a keratan sulfate oligosaccharide. As to the properties of the solution of L4, it was a colorless transparent aqueous solution having a pH of about 6 to 7 and an osmotic pressure ratio to physiological saline of about 1.

### 〈Experimental Method〉

10-week-old male MRL (lpr/lpr) mice (Izui S., et al., Mechanism of genetic control of murine systemic lupus erythematosus., In Systemic Lupus Erythematosus, pp. 3-12, Ed. Peter A. Miescher, Springer-Verlag Berlin, 1995) and male C57BL/6 mice as a control were used. The group constitution of the mice is shown below. The grouping was performed in principle one day prior to the day when the administration of test substances was started. Blood was collected from the orbital venosus plexus of the animals during their preliminary feeding period and they were grouped so that there was no substantial difference in average value and standard deviation between the resulting groups using blood creatinine level (CRE) and blood urea nitrogen level (BUN) as indices. Also, it was set so that the standard deviation became as small as possible.

**Table 1**

| Group No. | Test substance | Mouse | L4 administered dose (mg/kg) | L4 administered concentration (mg/ml) | L4 administered volume (ml/kg) | Number of mice, male |
|---|---|---|---|---|---|---|
| 1 | PBS | MRL | - | - | 2.5 | 7 |
| 2 | L4 | MRL | 2.5 | 1 | 2.5 | 7 |
| 3 | L4 | MRL | 5 | 2 | 2.5 | 7 |
| 4 | L4 | MRL | 10 | 4 | 2.5 | 7 |
| 5 | Untreated | C57BL/6 | - | - | - | 5 |

| | | | | | | |
|---|---|---|---|---|---|---|
| PBS designates phosphate-buffered physiological saline. | | | | | | |

A PBS solution of test substance was administered intramuscularly to animals for 35 days continuously during the 11 to 16 weeks old. After completion of the administration, blood was collected from the posterior aorta. The collected blood was subjected to physiochemical autoanalyzer (COBAS MIRA S; marketed by Nippon Rosche) and CRE was measured.

Using the same group constitution (each group consisting of 12 mice), the administration was Performed in the same manner as described above, and the kidney was excised and pathological and histological tests on glomerulus and renal artery were performed.

The glomeruli were grouped into two types, i.e., (1) one having an image of glomerulus, showing very slight thickening of ansae and cell proliferation, which is judged to fall into the type Minimal Change (MC) according to the classification of WHO and (2) one having an image of glomerulus, showing almost complete disappearance of the cavity of Bowman's capsule or of blood vessels and the glomerulus being fully stuffed with cell components or precipitates, so that its function is presumed to be completely lost (the image of glomerulus corresponding to Proliferative Glomerulonephritis of Diffuse Glomerulonephritis (DP) according to the classification of WHO). The numbers of glomeruli showing MC and DP were counted, respectively, and the count numbers were divided by the total number of glomeruli to calculate their incidence(%).

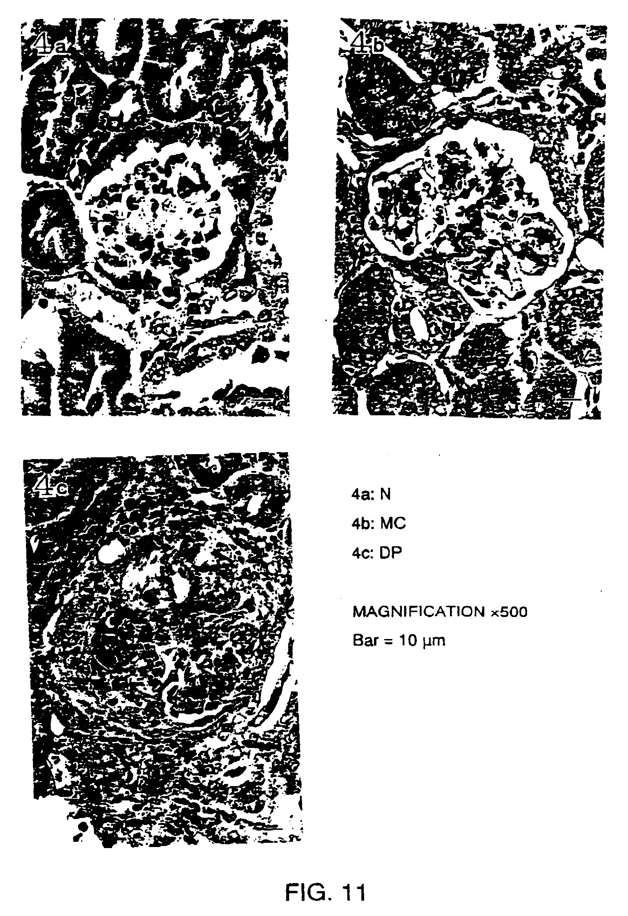

Fig. 11 is a set of photographs illustrating images of normal glomeruli (upper left), MC glomeruli (upper right), and DP glomeruli (lower left) (magnification: ×500, the bar at lower rignt in each photograph indicates 10 µm).

Fig. 11 suggests that the lesion in glomeruli changes gradually from MC to the state where the cavity of Bowman's capsule or blood vessels disappeared due to proliferation of monocytes or mesangial cells or precipitation of fibrin and immune complexes, i.e., DP. In other words, it can be seen that MC is closer to normal than DP, and DP is more suffered from fibrosis than MC.

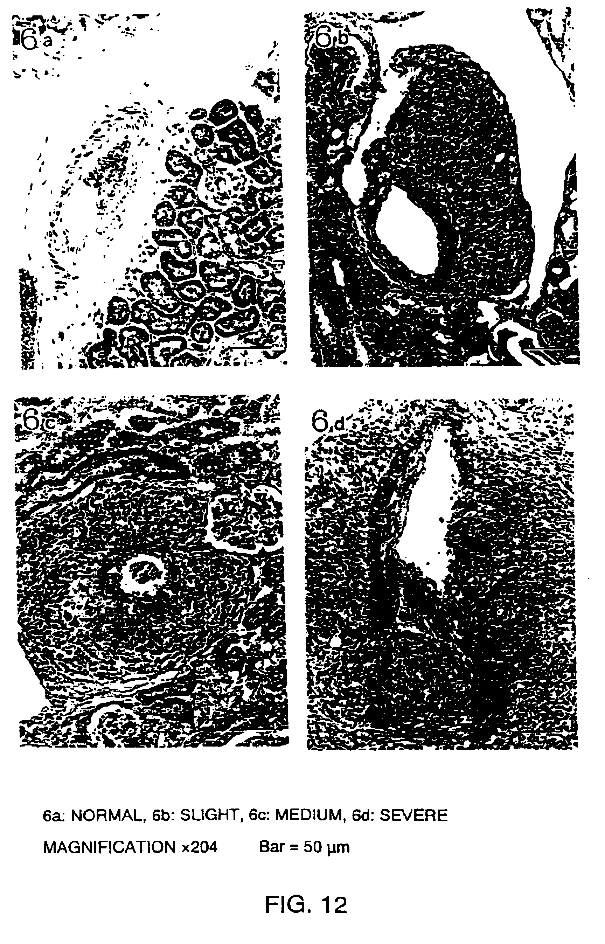

Tissue images of renal artery were ranked according to the following grades 1 to 4 and each of their incidence was counted. Fig. 12 (at a magnification of ×204, with the bar at lower right in each photograph indicating 50 µm) illustrates respective images of grades 1 to 4.
Grade 1: Almost normal renal artery image (upper left).
Grade 2: Infiltration of inflammatory cells around the blood vessels is observed (upper right).
Grade 3: Infiltration of inflammatory cells around the blood vessels and ruin of tunica adventitia and tunica media (degeneration of vacuoles and precipitation of fibrin) are observed (lower left).
Grade 4: Infiltration of inflammatory cells around the blood vessels, ruin of tunica adventitia and tunica media (degeneration of vacuoles, precipitation of fibrin and plasia of smooth muscle cells) are observed, and infiltration of inflammatory cells into tunica interna and tunica media are observed (lower right).

Grade 1 is closest to normal and Grade 4 shows the fibrosis accompanying angiitis to the furthest extent.

### 〈Results〉

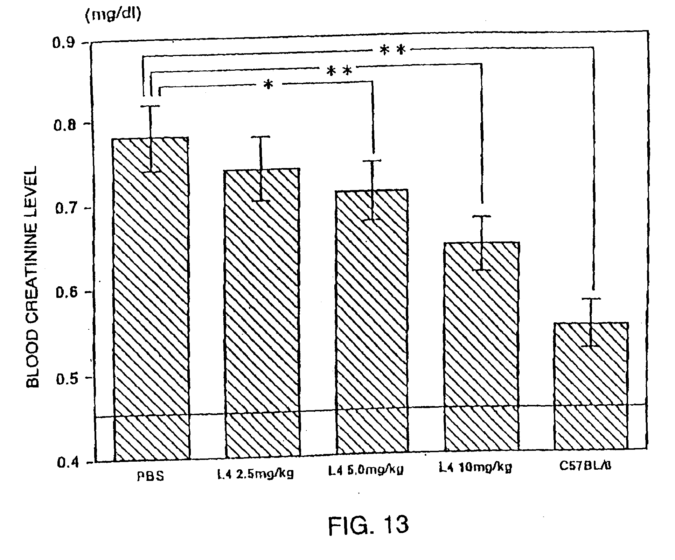

A significant decrease in blood creatinine level (CRE) was observed in each L4-administered group (Group Nos. 2 to 4) (Fig. 13). This shows that administration of L4 brings the renal function in a trend of recovery, suggesting inhibition of fibrosis.

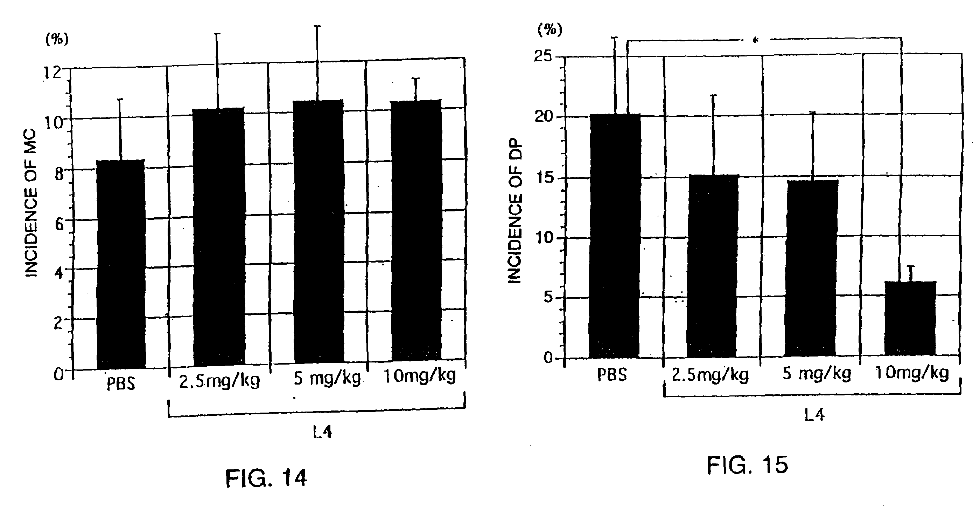

The incidence of MC in glomeruli increased in each L4-administered group (Group Nos. 2 to 4) as compared with the PBS-administered group (Group No. 1) (Fig. 14). The incidence of DP in glomeruli decreased in each L4-administered group (Group Nos. 2 to 4) as compared with the PBS-administered group (Group No. 1). In particular, a significant decrease was observed in the group administered with 10 mg/kg of L4 (Group No. 4) (Fig. 15). These results show that L4 has an antifibrotic activity.

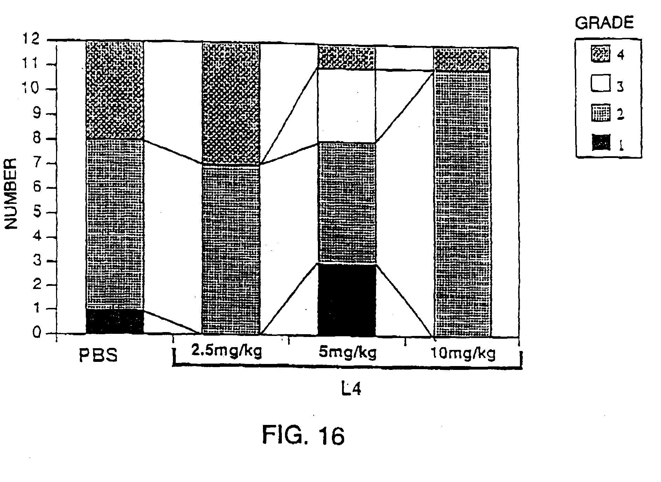

Regarding the tissue image of renal artery, particularly Grade 4 decreased considerably in the groups in which L4 was administered in amounts of 5 mg/ml (Group No. 3) and 10 mg/ml (Group No. 4) and tissue images closer to normal were observed (Fig. 16). This also shows that L4 has an antifibrotic activity.

Note that in Fig. 13, the symbol "*" indicates that the values were significant at a level of p<0.05 and the symbol "**" indicates that the values were significant at a level of p<0.01 according to the multiple comparison test of Williams. Also, in Fig. 15, the symbol "*" indicates that the values were significant at a level of p<0.05 according to the multiple comparison test of Bonferroni.

### Test Example 3: Study of effects on diabetic nephropathy in spontaneous diabetic mice (db/db)

In the drug effect and pharmacological test example 3 were used sodium salt of L4 was used as a keratan sulfate oligosaccharide. After dissolving sodium salt of L4 to a concentration of 6% (w/v) in distilled water for injection, the resulting solution was diluted to a predetermined concentration with physiological saline prior to use. As a positive control was used captopril (ACE inhibitor; manufactured by Sankyo Co., Ltd.). Captopril was dissolved in distilled water for injection to a predetermined concentration prior to use.

### 〈Experimental Method〉

C57BL (db/db) mice (female mice of 34 weeks old) that were suffered from diabetes and that excreted urine albumin in amounts of 2,700 to 6,000 µg or more in 24 hours were used for experimentation. Also, healthy C57BL mice (female mice of 10 weeks old) were used. The group constitution of mice was as follows.

**Table 2**

| Group name | Test substance | Dose (mass/volume /weight) | Method of administration | Number of mice, female |
|---|---|---|---|---|
| Control | Physiological saline | 2 ml/kg | Subcutaneous at the back | 10 |
| L4-administered | L4 | 5 mg/2 ml/kg | Subcutaneous at the back | 10 |
| L4-administered | L4 | 10 mg/2 ml/kg | Subcutaneous at the back | 10 |
| L4-administered | L4 | 20 mg/2 ml/kg | Subcutaneous at the back | 10 |
| Positive control | Captopril | 3 mg/10 ml/kg | Oral | 10 |
| Normal | - | - | - | 10 |

Each test substance was administered subcutaneously at the back of animals in blind 6 times a week for 8 weeks. As the positive control, captopril was similarly administered 6 times a week for 8 weeks by oral administration.

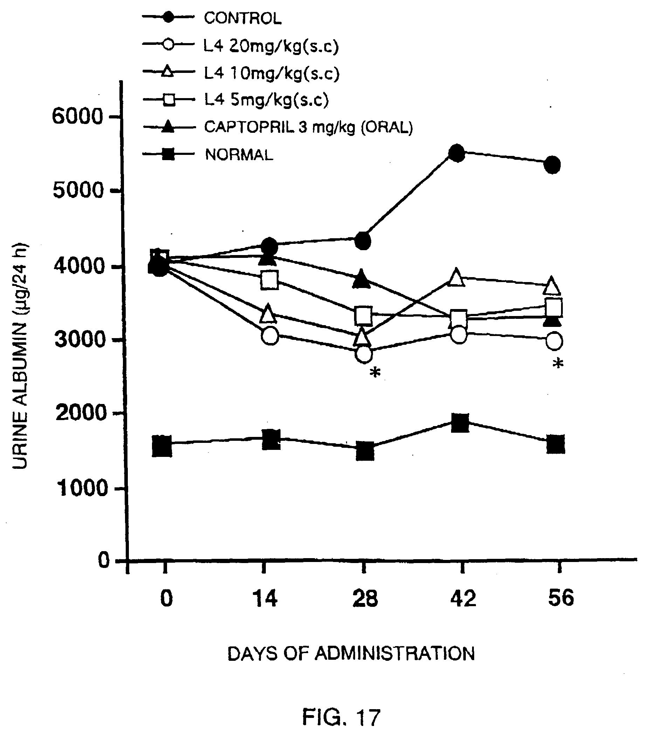

At the time of grouping and every 2 weeks after the initiation of the administration of the drug, urine was accumulated in a metabolic cage for 24 hours. After measuring the amount of urine, the urine albumin concentration was measured by use of Micro Albumin HA Test Wako (manufactured by Wako Pure Chemical Industries Ltd.) and the amount of urine albumin secreted in 24 hours was calculated from the urine albumin concentration and the amount of urine. Fig. 17 illustrates the results.

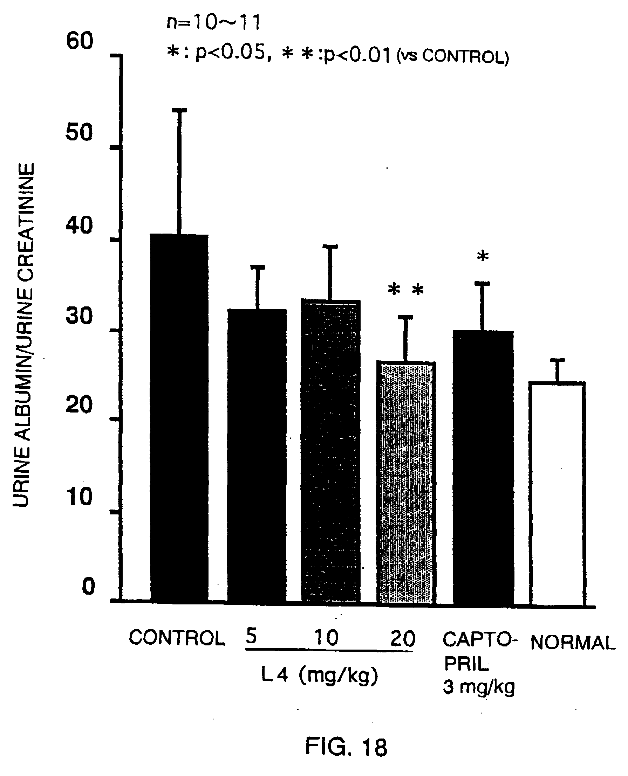

After 8 weeks from the administration of the drug, the urine creatinine concentration was measured using a physiochemical autoanalyzer (COBAS MIRA S; marketed by Nippon Rosche) and the ratio of the urine albumin concentration to urine creatinine concentration was calculated. Fig. 18 shows the results.

Note that in Figs. 17 and 18, the symbol "*" indicates that the values were significant at a level of p<0.05 and the symbol "**" indicates that the values were significant at a level of p<0.01 relative to the control in the multiple comparison test of Dunnett.

### 〈Results〉

For the control group, a clear increase in urine albumin level was observed and it was also evident that in the control group, the amount of albumin secreted increased with lapse of time (Fig. 17).

For the L4-administered groups, the urine albumin level started to decrease after 2 weeks from the administration and thereafter all the test groups showed lower values than the control group. After 4 weeks and 8 weeks, a significant inhibitory effect was observed in the group of L4 20 mg/kg.

For the positive control group, the value started to decrease after 4 weeks after the administration and thereafter not significant but lower values were shown (Fig. 17).

Regarding the ratio of urine albumin level/urine creatinine level after 8 weeks from the administration, L4-administered groups all showed lower values than the control group and a significant inhibition was observed in the group of L4 20 mg/kg. A significant inhibitory effect was observed for the positive control group as compared with the control group (Fig. 18).

In the case of diabetic nephropathy, it has revealed that generally the urine albumin level gradually increases according to the pathema of a disease. In this model, an increase in urine albumin level with lapse of time was similarly observed, which showed that the pathema gradually progressed.

L4 quickly inhibited secretion of albumin in urine and improved. The inhibitory effect of L4 appeared earlier than in the positive control group, which suggests that L4 is possibly more useful than the ACE inhibitory drug.

### Preparation Examples

### 1. Injectable preparation

In a conventional manner, L4 was dissolved at a final concentration of 10 mg/ml in physiological saline that had been adjusted to pH 6.8 to 7.6 with a phosphate, and the solution was sterilized by filtration, followed by dispensing it in a vial in an amount of 1 ml and sealing to produce an injectable preparation.

### (2) ointment

L4L4 was dissolved at a final concentration of 10 mg/ml in a hydrophilic ointment prescribed in the Pharmacopoeia of Japan to produce an ointment.

### (3) Spray

In a conventional manner, L4 was dissolved at a final concentration of 10 mg/ml in physiological saline that had been adjusted to pH 6.8 to 7.6 with a phosphate, and the solution was sterilized by filtration, followed by dispensing it in a vial in an amount of 1 ml and sealing to produce a spray.

## Claims

1. An antifibrotic agent comprising a keratan sulfate oligosaccharide or a pharmaceutically acceptable salt thereof as an active ingredient.

2. The antifibrotic agent according to claim 1, which is a prophylactic or a depressant for fibrosis.

3. The antifibrotic agent according to claim 2, wherein the agent is used as a prophylactic or a depressant for fibrosis accompanying one or more diseases selected from the group consisting of pulmonary fibrosis, hepatocirrhosis, arteriosclerosis, scleroderma, restenosis of coronary artery after percutaneous transluminal coronary angioplasty, interstitial myocarditis, interstitial cystitis, glomerulonephritis, angiitis, diabetic nephropathy, hypertensive nephrosclerosis, HIV nephropathy, IgA nephropathy, lupus nephritis, interstitial nephritis, obstructed kidney due to ureteral obstruction, skin scar formation after burn, and toxicosis.

4. The antifibrotic agent according to any one of claims 1 to 3, wherein the fibrosis is a fibrosis caused by administration of a drug.

5. The antifibrotic agent according to claim 4, wherein the drug is an antineoplastic agent, an antibiotic, an antibacterial agent, an antiarrhythmic agent, an antiphlogistic agent, an antirheumatic agent, an interferon, or shosaikoto.

6. The antifibrotic agent according to claim 4, which is a prophylactic or a depressant for pulmonary fibrosis accompanying administration of an antineoplastic agent.

7. The antifibrotic agent according to claim 6, wherein the antineoplastic agent is an antineoplastic antibiotic.

8. Use of a keratan sulfate oligosaccharide or a pharmaceutically acceptable salt thereof for the manufacture of an antifibrotic agent.
